(19) Europäisches Patentamt
European Patent Office
Office européen des brevets

(11) **EP 3 451 921 B1**

(12) **EUROPEAN PATENT SPECIFICATION**

(45) Date of publication and mention
of the grant of the patent:
**08.09.2021 Bulletin 2021/36**

(51) Int Cl.:
*A61B 5/08* *(2006.01)*    *A61M 15/00* *(2006.01)*
*A61M 16/00* *(2006.01)*    *A61M 16/01* *(2006.01)*
*A61M 16/08* *(2006.01)*    *A61M 16/10* *(2006.01)*
*A61M 16/18* *(2006.01)*    *A61M 16/20* *(2006.01)*
*A61M 16/22* *(2006.01)*

(21) Application number: **17793459.3**

(22) Date of filing: **05.05.2017**

(86) International application number:
**PCT/US2017/031322**

(87) International publication number:
**WO 2017/193007 (09.11.2017 Gazette 2017/45)**

(54) **SYSTEMS AND METHODS FOR SENSING INHALATIONAL ANESTHETIC AGENTS**

SYSTEME UND VERFAHREN ZUR ERFASSUNG INHALATIVER ANÄSTHESIEMITTEL

SYSTÈMES ET PROCÉDÉS DE DÉTECTION D'AGENTS ANESTHÉSIQUES PAR INHALATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.05.2016 US 201662332878 P**

(43) Date of publication of application:
**13.03.2019 Bulletin 2019/11**

(73) Proprietor: **University of Utah Research Foundation
Salt Lake City, UT 84108 (US)**

(72) Inventors:
• **KUCK, Kai**
  **Park City**
  **UT 84098 (US)**
• **ORR, Joseph, A.**
  **Park City**
  **UT 84098 (US)**
• **KOLBAY, Patrick, R.**
  **Salt Lake City**
  **UT 84108 (US)**

(74) Representative: **CH Kilger Anwaltspartnerschaft mbB
Fasanenstraße 29
10719 Berlin (DE)**

(56) References cited:
EP-A1- 0 545 567      WO-A2-2016/065265
US-A1- 2006 124 127    US-A1- 2006 260 612
US-A1- 2010 168 599    US-A1- 2011 105 934
US-A1- 2012 031 402    US-A1- 2012 312 302
US-A1- 2015 209 546    US-A1- 2015 209 546
US-B1- 6 470 741

**Description**

**STATEMENT OF GOVERNMENT SUPPORT**

This invention was made with government support under Grant Number 56000234 NNX15A124H awarded by the National Aeronautics and Space Administration. The government has certain rights in this invention.

**FIELD**

This invention relates to systems for sensing inhalational anesthetic agents, conserving anesthetic agents, and, in exemplary aspects, to systems for determining the concentration of inhalational anesthetic agents within a breathing circuit, as well as systems for conserving anesthetic gases.

**BACKGROUND**

Measuring the concentration of volatile anesthetics is important in anesthesia care. Current technology uses side stream infrared analysis to determine anesthetic agent concentration with high accuracy, but this technique is cost prohibitive in both small practice environments and low resource areas.

Anesthesiologists in low-income and low-resource areas are often challenged by the lack of medical equipment available. Many developing countries rely on donor aid, with upwards of 80% of the medical equipment provided by international donors and foreign governments. However, due to the inherent complexity of anesthetic equipment, maintenance and repair is difficult and results in as little as 10% of devices becoming operational. *See* Gatrad AR et al. Anaesthesia. 2007. vol. 62, 90-95.

The following documents also disclose systems for determining an anaesthetic gas concentration to be delivered to a patient: US 2011/105934, EP 0545567, WO 2016/065265, US 2015/209546.

Thus, there is a need for low-cost, robust, and portable anesthesia delivery systems that are suited for these environments and that are capable of accurately and reliably determining the concentration of volatile anesthetic gas in a rebreathing circuit.

**SUMMARY**

The invention is described in independent claim 1. Described herein, in various examples, are systems and methods for sensing inhalational anesthetic agents within a breathing circuit. An exemplary system can have a vaporizer configured to deliver a gaseous anesthetic agent to the breathing circuit; a blower assembly configured to circulate gas within the breathing circuit; and a flow sensing assembly configured to produce a measurement signal indicative of the concentration of the gaseous anesthetic agent within the breathing circuit. An exemplary method can include delivering a gaseous anesthetic agent to a breathing circuit; circulating gas within the breathing circuit; and using a flow sensing assembly to produce a measurement signal indicative of the concentration of the gaseous anesthetic agent within the breathing circuit. Optionally, the system can have a reflector assembly for capturing anesthetic agent and then selectively returning the captured anesthetic agent to a breathing circuit as disclosed herein.

**DESCRIPTION OF THE FIGURES**

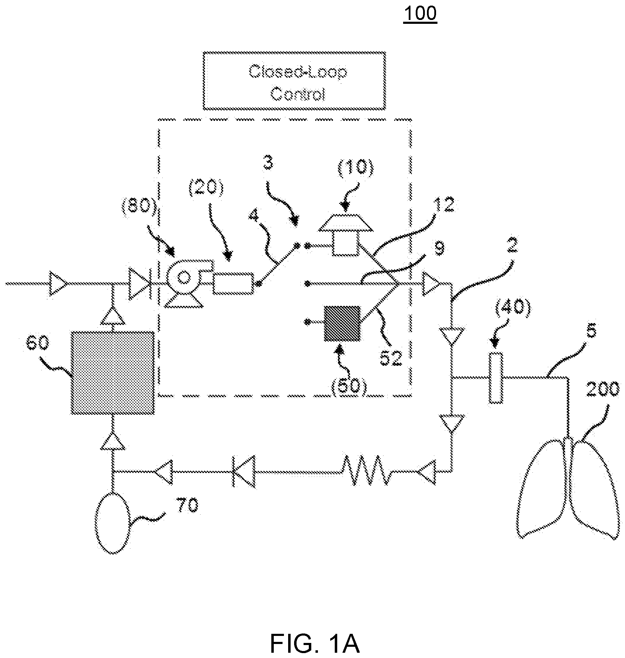

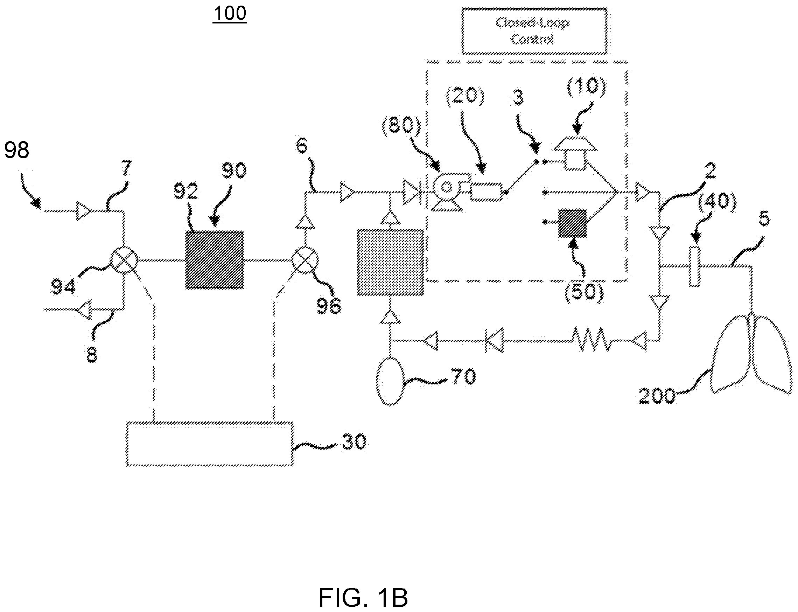

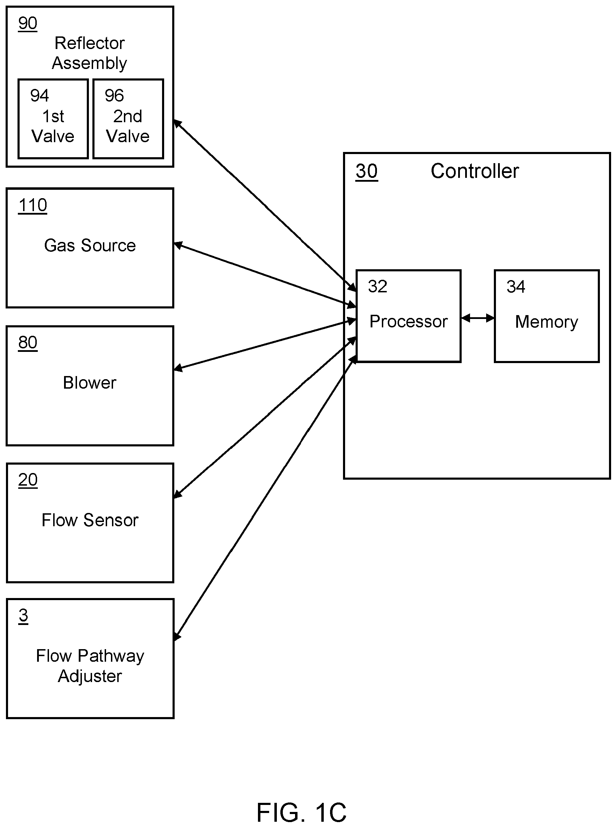

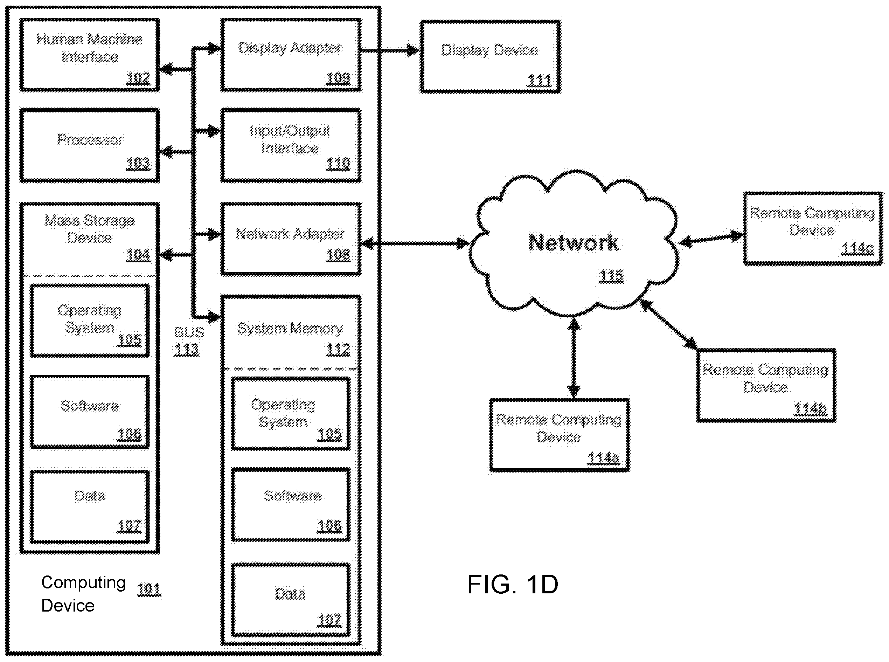

FIG. 1A is a schematic diagram depicting an exemplary system for sensing inhalational anesthetic agents as disclosed herein. FIG. 1B is a schematic diagram depicting another exemplary system for sensing inhalational anesthetic agents as disclosed herein. FIG. 1B differs from FIG. 1A in that the system depicted in FIG. 1B includes a charcoal reflector for recycling (capturing and reflecting) anesthetic agents for further use by the system. FIG. 1C is a schematic diagram depicting an exemplary arrangement of the controller and other components of the system that can be controlled by the controller. FIG. 1D is a schematic diagram depicting an exemplary controller provided in the form of a computing device.

FIG. 2 is a transparent perspective view of an exemplary flow sensor as disclosed herein.

FIG. 3 is a graph comparing an actual anesthetic gas concentration measured by a gas analyzer to an estimated anesthetic gas concentration determined by a system comprising a combined thermal and differential pressure sensor as disclosed herein.

FIG. 4 is another graph comparing an actual anesthetic gas concentration measured by a gas analyzer to an estimated anesthetic gas concentration determined by a system comprising a combined thermal and differential pressure sensor as disclosed herein.

FIG. 5 is a graph showing the observed concentration of isoflurane leaving the vessel containing 40 grams of saturated activated charcoal as the flow was reversed at 2 liters per minute as disclosed herein. As shown, the activated charcoal (black) allowed for the gradual release of isoflurane compared to the control (grey), which contained no activated charcoal.

FIG. 6 is a graph showing the observed concentration of isoflurane during ventilation between the vessel of 10 grams of activated charcoal and the test lung as disclosed herein. Similar to FIG. 5, the activated charcoal (dark grey) allowed for the gradual release of isoflurane compared to the control (light grey), which contained no activated charcoal. A running average is shown for both the activated charcoal (black) and the control (grey).

FIG. 7 is a schematic diagraph depicting an exemplary system comprising a combined differential pressure and thermal flow sensor in conjunction with a radial blower as disclosed herein.

FIG. 8 is a cross-sectional side view of a flow sensor comprising a bypass channel. As shown, a thermal flow sensor can be situated within the bypass channel.

FIG. 9 is a graph comparing an anesthetic gas concentration measured by an infrared spectroscopy gas analyzer to an anesthetic gas concentration determined by a system comprising a combined differential pressure and thermal flow sensor as disclosed herein.

## DETAILED DESCRIPTION

The present invention can be understood more readily by reference to the following detailed description, examples, drawings, and claims. However, before the present devices, systems, and/or methods are disclosed and described, it is to be understood that this disclosure is not limited to the specific devices, systems, and/or methods disclosed unless otherwise specified, as such can, of course, vary. It is also to be understood that the terminology used herein is for the purpose of describing particular aspects only and is not intended to be limiting.

The following description of the invention is provided as an enabling teaching of the invention in its best, currently known embodiment. To this end, those skilled in the relevant art will recognize and appreciate that many changes can be made to the various aspects of the invention described herein, while still obtaining the beneficial results of the present invention. It will also be apparent that some of the desired benefits of the present invention can be obtained by selecting some of the features of the present invention without utilizing other features. Accordingly, those who work in the art will recognize that many modifications and adaptations to the present invention are possible and can even be desirable in certain circumstances and are a part of the present invention. Thus, the following description is provided as illustrative of the principles of the present invention and not in limitation thereof.

As used throughout, the singular forms "a," "an" and "the" comprise plural referents unless the context clearly dictates otherwise. Thus, for example, reference to "an anesthetic agent" can comprise two or more such anesthetic agents unless the context indicates otherwise.

Ranges can be expressed herein as from "about" one particular value, and/or to "about" another particular value. When such a range is expressed, another aspect comprises from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

As used herein, the terms "optional" or "optionally" mean that the subsequently described event or circumstance can or cannot occur, and that the description comprises instances where said event or circumstance occurs and instances where it does not.

The word "or" as used herein means any one member of a particular list and also comprises any combination of members of that list.

As used herein, the terms "line" and "circuit" are indicative of structures that permit gas flow as disclosed herein. Thus, it is contemplated that such "lines" and "circuits" can comprise tubing, conduits, valves, couplings, and the like that are conventionally used for permitting gas flow as disclosed herein.

Modern inhalational anesthetic machines are designed to provide a variety of functions in the administration of anesthesia - delivery of fresh gas flow of oxygen and anesthetic vapor to a patient through a breathing circuit, sensing and monitoring of these gas concentrations, and ventilating the patient either though spontaneous, manual, or mechanical means. An ideal anesthesia machine would allow for instantaneous control and manipulation of these parameters, with little to no risk to the patient. Sensing and delivery of anesthetic agents has greatly improved, allowing for highly controllable systems. Still, anesthetic equipment remains cost- and resource-prohibitive for many applications. Thus, there remains a need for compact, efficient, and low cost anesthesia administration technologies that retain the precision and accuracy of current methods.

Accurate dosing of anesthetic vapors is the central function of any anesthetic machine and fundamental to the practice of anesthesia. When anesthetics are delivered inaccurately or inappropriately, adverse side effects can lead to inadequate sedation, postoperative complications, or even mortality. Most modern anesthetics are liquid at room temperature but are highly volatile, allowing for easy vaporization and subsequent inhalation. Delivery of these anesthetics is achieved via controlled vaporization into a carrier gas, most often oxygen. Because different anesthetic agents have varying physiological and physical characteristics, controlled dosing is challenging. To address the issues of varying physiological effects of each anesthetic agent, the notion of minimum alveolar concentration (MAC) was introduced to standardize anesthetic potency. Specifically, MAC is the exhaled concentration of anesthetic gas needed to prevent patient movement in response to a skin incision on 50% of the population.

Expired concentration of anesthetic is one of the best measurable indicators of how deeply a patient is anesthetized as it directly correlates to the concentration in the body. However, this still leaves the challenge of varying physical properties of each anesthetic, notably the differing vapor pressure. As a result of this, anesthetic machines typically contain multiple vaporizers that are calibrated and designed for the different properties of each gas, allowing for the specific titration of each individual anesthetic. This, in and of itself, causes a large amount of redundancy in anesthetic machines making them incredibly large and bulky.

Despite the fact that specialized anesthetic vaporizers are needed for every unique agent, changes in pressure, flow rates, and temperature can still affect vaporization. This results in discrepancies of set delivered concentrations and actual delivered concentrations. Monitoring the inspiratory concentration of anesthetic is critical to ensure an appropriate and safe amount of anesthetic is being delivered to the patient. Measuring the expiratory concentration also helps to determine the current depth of anesthesia. Monitoring oxygen and carbon dioxide levels is also required during anesthetic maintenance. Oxygen concentration needs to be sustained above a certain level and monitored to avoid delivering hypoxic mixtures to the patient. Carbon dioxide must also be measured to determine pulmonary perfusion, alveolar ventilation, respiratory patterns, and appropriate elimination of carbon dioxide from the breathing circuit. The current technology uses side stream infrared analysis to determine anesthetic agent and carbon dioxide concentration with high accuracy as each anesthetic gas absorbs different wavelengths of infrared light. Oxygen is sensed using galvanic, polarographic, or paramagnetic techniques. The paramagnetic method is most commonly used in modem anesthesia devices, and takes advantage of interactions between the free electron pair in oxygen and magnetic fields to determine concentration.

Another side effect of anesthetic drugs is depressing the patient's respiratory drive and ultimately causing apnea, a fatal condition if left untreated. Ventilators are required to mechanically move gases, including oxygen and anesthetic vapors, into the lungs and remove carbon dioxide from the lungs. Most anesthetic machines contain automatic ventilators to relieve the anesthesiologist from having to physically squeeze manual ventilators. Gases are forced into the lungs by bellows, pistons, or blowers creating changes in pressure. Various parameters can be controlled in these ventilator systems, including tidal volume, positive end-expiratory pressure (PEEP), and respiratory rate. These parameters are adjusted and controlled through a combination of pressure and flow sensors. Smart technologies in these ventilators allow for detection in attempted spontaneous breathing in patients who are not fully apneic. Ventilators are designed to support patients' spontaneous breathing efforts and to match patient's natural physiological respiratory drive.

With the incorporation of feedback control into anesthesia machines, the limiting factor is most often the accuracy and precision of the gas sensor. The gas analysis components also constitute some of the more expensive features in anesthetic machines.

Described herein with reference to FIGS. 1A-2 are systems and methods for sensing inhalational anesthetic agents. In use, the disclosed systems can be employed in low-cost anesthesia machines, which can optionally be used in office-based environments and/or in low-resource environments.

As further disclosed herein, flow sensors that measure a multitude of physical and chemical property differences between anesthetic gases, oxygen, and carbon dioxide can provide opportunities for measuring gas concentration based on property differences beyond those that are conventionally used. In various aspects, it is contemplated that a thermal sensor can be used to measure gas flow. For example, it is contemplated that a hot wire anemometer can use convective heat transfer to measure gas flow. In this example, as fluid flows across a heated resistive wire, the filament is cooled, with higher fluid velocities yielding increased cooling. Therefore, measurement of the required voltage to maintain a constant temperature in the filament is directly proportional to the fluid velocity. Given a known flow rate, hot-wire anemometry (and other thermal sensors) can also be used to determine the concentration of fluids with different heat capacities. *See* Libby, P. A., Way, J. (1970) "Hotwire probes for measuring velocity and concentration in helium-air mixtures" AIAA Journal 8(5): 976-978. As further disclosed herein, it is contemplated that the use of thermal sensors, such as hot-wire anemometers, to measure anesthetic agent concentrations inline can yield an inexpensive, simple, and compact alternative to infrared analysis of gases. Although hot-wire anemometers are specifically disclosed as an example of a thermal sensor, it is contemplated that other thermal sensors can be used. However, it is understood that the principles of operation of such thermal sensors can be generally the same as those of the hot-wire anemometer; that is, the thermal sensors can rely on the rate of heat being pulled off of a filament and correlate that rate to other parameters of a liquid or gas, such as velocity, heat coefficient, and the like.

In addition, or in the alternative, to the use of a thermal sensor, it is contemplated that the disclosed system 100 can further comprise a differential pressure sensor. In use, it is contemplated that flow measured by differential pressure through on orifice can be sensitive to changes in anesthetic concentration. Specifically, because of the disparity in density of anesthetic gases to their carrier gases (nitrogen, oxygen, carbon dioxide, water vapor, etc) the influence on the differential pressure can vary significantly based on concentration due to Bernoulli's principle.

In exemplary aspects, as shown in FIGS. 1A-1B, an anesthesia system 100 can define a breathing circuit 2, which, as further disclosed herein, can be configured to provide closed-loop control of the delivery of gaseous anesthetic agents. In these aspects, the anesthesia system 100 can comprise a vaporizer 10 configured to deliver a gaseous anesthetic agent to the breathing circuit 2. The vaporizer 10 can be a conventional vaporizer as is known in the art for delivering anesthetic agents to a breathing circuit. Thus, in some aspects, the vaporizer 10 can rely on a "drawover" technique as is known the art. In these aspects, it is contemplated that the vaporizer 10 can comprise a bypass valve that is configured to direct incoming fresh oxygen over a liquid anesthetic, which then quickly evaporates. Alternatively, in other aspects, the vaporizer 10 can use an injector-type design (e.g., a direct liquid injection vaporizer design) to deliver anesthetic agents to the breathing circuit 2.

In another aspect, the anesthesia system can comprise a blower assembly 80 configured to circulate gas within the breathing circuit. Optionally, it is contemplated that the blower assembly can comprise a motorized blower (e.g., a turbine blower) as is known in the art. However, it is contemplated that any conventional means for circulating gas within a breathing circuit can be used.

In further aspects, and with reference to FIGS. 1A-2, the anesthesia system 100 can comprise a flow sensing assembly 20, which can comprise a thermal sensor (e.g., a hot-wire anemometer), a differential pressure sensor, or combinations thereof. The flow sensing assembly 20 can be configured to produce a measurement signal (optionally, a plurality of measurement signals) indicative of the concentration of the gaseous anesthetic agent within the breathing circuit 2. Although specific sensors are described herein, it is contemplated that the flow sensing assembly 20 can comprise other sensors that are likewise capable of producing a measurement signal as disclosed herein. In use, and as further disclosed herein, the sensor(s) of the flow sensing assembly 20 can be calibrated to a base flow rate as further disclosed herein, and as an anesthetic agent is delivered to the breathing circuit 2, any change in the output of the sensor(s) of the flow sensing assembly can be indicative of a change in flow rate as a result of the added anesthetic agent.

In additional aspects, the anesthesia system 100 can comprise processing circuitry 32 that is communicatively coupled to the flow sensing assembly 20. In these aspects, the processing circuitry 32 can be configured to receive the measurement signal(s) produced by the flow sensing assembly and to determine the concentration of the gaseous anesthetic agent within the breathing circuit 2. Optionally, the processing circuitry 32 can be provided as the processing unit of a controller 30. In exemplary aspects, the processing circuitry 32 (e.g., processing unit) of the controller 30 can be communicatively coupled to a memory that stores program instructions, modules, data, and the like that can be retrieved to permit performance of the processing circuitry 32 as disclosed herein. Optionally, the controller 30 can be provided as a computing device 101 as further disclosed herein.

In exemplary aspects, the flow sensing assembly 20 can comprise a thermal sensor 22. In these aspects, the thermal sensor 22 can be configured to produce a measurement signal indicative of a change in temperature within the breathing circuit 2. In these aspects, the processing circuitry 32 of the controller can be configured to correlate the measured change in temperature to a change in the velocity of gas flow and to the concentration of the gaseous anesthetic agent within the breathing circuit. Optionally, in some aspects, the thermal sensor can comprise a hot-wire anemometer having a heated resistive wire. In these aspects, the hot-wire anemometer can be configured to produce a measurement signal indicative of a voltage required to maintain a temperature of the heated resistive wire as gas in communication with the flow sensor flows within the breathing circuit 2. It is contemplated that the processing circuitry 32 can be configured to correlate the required voltage to a change in the velocity of gas flow and to the concentration of the gaseous anesthetic agent within the breathing circuit.

In further exemplary aspects, the flow sensing assembly 20 can comprise a differential pressure sensor 24. In these aspects, the differential pressure sensor can be configured to produce a measurement signal indicative of a change in pressure within the breathing circuit 2. It is contemplated that the processing circuitry 32 can be configured to correlate the measured change in pressure to a change in the velocity of gas flow within the breathing circuit 2 and to the concentration of the gaseous anesthetic agent within the breathing circuit.

According to the invention the flow sensing assembly 20 comprises both a thermal pressure sensor 22 and a differential pressure sensor 24. The thermal and differential pressure sensors are configured to produce at least one measurement signal indicative of changes in temperature and pressure within the breathing circuit. The processing circuitry is configured to correlate the measured changes in temperature and pressure to a change in the velocity of gas flow and to the concentration of the gaseous anesthetic agent within the breathing circuit.

As shown in FIGS. 1A-1B, it is contemplated that the anesthesia system 100 can further comprise a patient line 5 positioned in fluid communication with the breathing circuit 2. In exemplary aspects, the patient line 5 can be located downstream of the vaporizer 10 (i.e., be positioned in fluid communication with the breathing circuit 2 at a location that is downstream of the vaporizer). In use, the patient line 5 can direct gas to the patient 200. Optionally, the patient line 5 can comprise an air-moisture exchanger 40 or heat-moisture exchanger as are known in the art.

In exemplary aspects, the anesthesia system 100 can further comprise a charcoal filter 50 positioned in fluid communication with the breathing circuit 2. Optionally, in these aspects, the charcoal filter 50 can be positioned between the vaporizer 10 and the patient line 5.

In further aspects, the anesthesia system 100 can further comprise a carbon dioxide scrubber 60 positioned within (in fluid communication with) the breathing circuit 2. Optionally, in these aspects, the carbon dioxide scrubber 60 can be positioned between the patient line 5 and the flow sensing assembly 20. Optionally, the breathing circuit 2 can comprise a bypass line (not shown) that is configured to allow gas flowing within the breathing circuit to selectively bypass the carbon dioxide scrubber 60. It is contemplated that the bypass line can be positioned in selective fluid communication with a fluid control valve positioned upstream of the carbon dioxide scrubber to selectively permit or restrict passage of gas through the carbon dioxide scrubber. It is further contemplated that the fluid control valve can be selectively moveable to a bypass position in which gas flow is diverted through the bypass line and does not pass through the carbon dioxide scrubber. In exemplary aspects, the processing circuitry 32 can be configured to selectively adjust the position of the fluid control valve to control the flow profile of gas within the breathing circuit.

Optionally, the anesthesia system 100 can further comprise a reservoir 70 positioned in fluid communication with the breathing circuit 2 between the patient line 5 and the carbon dioxide scrubber 60. In exemplary aspects, the reservoir 70 can comprise a reservoir bag as is known in the art.

In exemplary aspects, the blower motor of the blower assembly can be communicatively coupled to the processing circuitry. Optionally, in other exemplary aspects, the blower assembly can be positioned upstream of the flow sensor.

In further aspects, and as shown in FIG. 1B, the anesthesia machine can further comprise a gas inlet-scavenge line 6 positioned in fluid communication with the breathing circuit 2. Optionally, in these aspects, the gas inlet-scavenge line 6 can be positioned between the carbon dioxide scrubber 60 and the flow sensing assembly 20 (i.e., the gas inlet-scavenge line can be positioned in fluid communication with the breathing circuit 2 at a location between the scrubber and the flow sensing assembly).

Optionally, in further exemplary aspects and as shown in FIG. 1B, the anesthesia system 100 can further comprise a reflector assembly 90 positioned in selective fluid communication with the breathing circuit 2. In these aspects, it is contemplated that the reflector assembly 90 can be positioned in selective fluid communication with the breathing circuit 2 through the gas inlet-scavenge line 6. In these aspects, the reflector assembly can comprise an anesthetic agent reflector 92 that is configured to capture gaseous anesthetic agent that exits the breathing circuit and to return the captured anesthetic agent to the breathing circuit. In one exemplary aspect, the anesthetic agent reflector 92 can optionally comprise activated carbon (e.g., charcoal); however, it is contemplated that any porous medium (e.g., zeolites, polymer matrices, and the like) can be selected for particular applications. Optionally, as shown in FIG. 1B, the reflector assembly 90 can be positioned between first and second fluid control valves 94, 96 positioned on respective downstream and upstream sides of the reflector assembly. Optionally, the second fluid control valve can be positioned between the anesthetic agent reflector 92 and the breathing circuit 2. In exemplary aspects, the first and second fluid control (flow control) valves 94, 96 can be communicatively coupled to the processing circuitry.

In exemplary aspects, the anesthesia system 100 can further comprise a gas inlet 7 positioned in fluid communication with the first fluid control valve 94. In these aspects, the gas inlet can be configured to receive fresh gas flow from a gas source 98. In further exemplary aspects, the anesthesia system 100 can comprise a scavenge outlet 8 positioned in fluid communication with the first fluid control valve 94. In these aspects, it is contemplated that the scavenger outlet 8 can receive discarded carbon dioxide and oxygen gas that passes through the anesthetic agent reflector 92 (moving away from the breathing circuit 2).

In use, the first and second fluid control valves 94, 96 can be moveable about and between a first position and a second position. In exemplary aspects, the processing circuitry can be configured to selectively move the first and second fluid control valves about and between the first position and the second position. In exemplary aspects, the first position of the first fluid control valve 94 can correspond to a gas-receiving position in which fresh gas flow from gas source 98 is allowed to pass through the first fluid control valve, and the second position of the first fluid control valve can correspond to a scavenge position in which fresh gas flow is blocked from entering the first fluid control valve but the first fluid control valve permits passage of gas into the scavenger outlet 8. In further exemplary aspects, the first position of the second fluid control valve 96 can correspond to an open position (that permits gas flow through the valve), and the second position of the second fluid control valve 96 can correspond to a closed position (that blocks gas flow through the valve). In exemplary aspects, the processing circuitry can be configured to move the first and second valves 94, 96 to define an open breathing circuit configuration, a closed breathing circuit configuration, or a partially closed breathing circuit configuration, wherein in the open breathing circuit configuration, the first and second valves are positioned in the first position, wherein in the partially open breathing circuit configuration, the first valve is positioned in the gas-receiving position and the second valve is positioned in the open position, and wherein in the closed breathing circuit configuration, the second valve is positioned in the closed position. With the breathing circuit in the partially open breathing circuit configuration, the anesthetic agent reflector 92 can collect anesthetic agent that passes through the reflector assembly (via gas inlet-scavenge line 6). After anesthetic agent is collected on the reflector 92, the processing circuitry can be configured to sequentially move the first and second valves from the partially closed breathing circuit configuration to the open breathing circuit configuration, thereby allowing for the flow of fresh gas to enter the reflector assembly 90 and guide the captured anesthetic agent back to the breathing circuit 2. Optionally, it is contemplated that the anesthesia system 100 can further comprise means for measuring or sensing saturation of the reflector 92. For example, in some optional aspects, the means for measuring or sensing saturation of the reflector can comprise a scale that is configured to weigh the reflector and to deliver an output to the processor 32 of the controller. In other optional aspects, the means for measuring or sensing saturation of the reflector can comprise a camera or another imaging device that is configured to capture an image of the reflector. In these aspects, it is contemplated that the camera can transmit captured images to the processor 32 of the controller 30 (or another, separate processing unit), which can then run imaging software to determine changes in the shape or appearance of the reflector 92 in comparison to a baseline image of the reflector before collection of anesthetic agent on the reflector. It is further contemplated that, using the processor, these changes in shape or appearance can be correlated to a corresponding quantity of anesthetic agent that has been collected on the reflector 92.

It is further contemplated that the anesthesia system can further comprise conventional equipment (valves, ports, and the like) for providing selective fluid communication between the breathing circuit and a subject or patient.

Optionally, the breathing circuit 2 can comprise a flow pathway adjuster 3 positioned between the flow sensing assembly 20 and the patient line 5 (downstream of the sensing assembly 20 but upstream of the patient line). As shown in FIGS. 1A-1B, in exemplary aspects, the breathing circuit 2 can comprise a plurality of flow lines that are in fluid communication with the patient line 5. In these aspects, the flow pathway adjuster 3 can be configured to provide selective fluid communication between the gas exiting the sensing assembly 20 and each of the respective flow lines. Optionally, the flow lines can comprise: a vaporizer line 12 that provides selective fluid communication between the gas exiting the sensing assembly 20 and the vaporizer 10; a filter line 52 that provides selective fluid communication between the gas exiting the sensing assembly and the charcoal filter 50; and a direct flow line 9 that permits direct fluid communication between the gas exiting the sensing assembly and the patient line 5. In exemplary aspects, the flow pathway adjuster 3 can comprise a valve assembly having at least one valve (optionally, a plurality of valves) that is moveable among a plurality of positions to selectively adjust the flow pathway of gas between the sensing assembly and the patient line 5. In these aspects, the valve assembly can be communicatively coupled to the processing circuitry 32, and the processing circuitry 32 can be configured to selectively adjust the position of each valve of the valve assembly to thereby produce the desired flow pathway arrangement. Alternatively, in other aspects, the flow pathway adjuster 3 can comprise an actuator that is communicatively coupled to the processing circuitry 32, with the processing circuitry being configured to selectively adjust the position (axial, rotational, angular, or combinations therof) of at least one of the flow lines or a portion of the patient line exiting the sensing assembly 20 to provide a desired flow pathway between the sensing assembly and the patient line 5.

In use, when additional anesthetic agent is needed (based upon, for example, the sensed concentration of anesthetic agent), it is contemplated that the flow pathway adjuster 3, in response to instructions from the processing circuitry 32, can effect fluid communication between the gas passing through the sensing assembly and the vaporizer. When too much anesthetic agent is present within the breathing circuit (based upon, for example, the sensed concentration of anesthetic agent), it is contemplated that the flow pathway adjuster 3, in response to instructions from the processing circuitry 32, can effect fluid communication between the gas passing through the sensing assembly and the charcoal filter, allowing the filter to reduce the anesthetic agent concentration. When the anesthetic agent is present at a desired or acceptable concentration (based upon, for example, the sensed concentration of anesthetic agent), the flow pathway adjuster 3, in response to instructions from the processing circuitry 32, can effect fluid communication between the gas passing through the sensing assembly and the direct flow line 9.

As further disclosed herein, it is contemplated that the anesthesia system can comprise at least one controller (e.g., computer, smartphone, tablet, programmable logic controller, and the like) for selectively controlling activation and/or performance of the various components of the system. For example, it is contemplated that a controller can be communicatively coupled to the blowing assembly for selective controlling the activation and/or performance parameters of the blowing assembly. In further exemplary aspects, it is contemplated that the hot-wire anemometer or other flow sensors of the flow sensing assembly can be communicatively coupled to the processor of the controller to receive outputs from the hot-wire anemometer or other flow sensors. In further exemplary aspects, when the system comprises a reflector assembly, it is contemplated that a controller can be provided to selectively open and close valves to permit or restrict fluid communication between the reflector assembly and the breathing circuit.

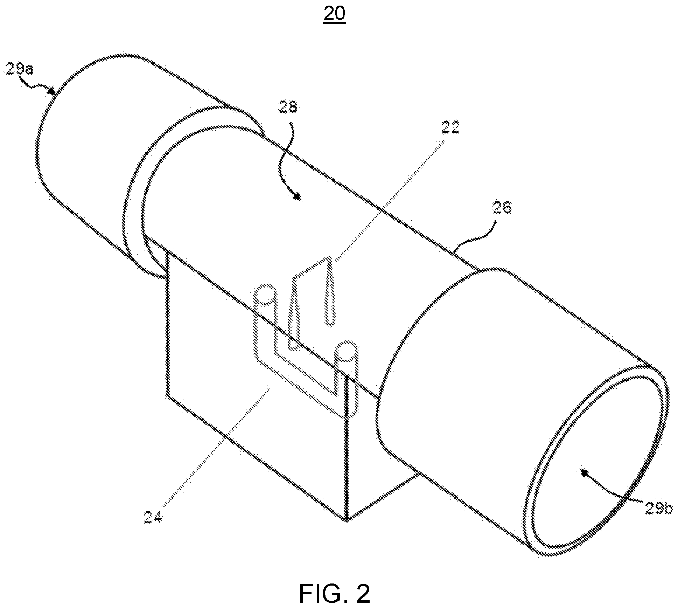

In further exemplary aspects, and with reference to FIG. 2, it is contemplated that the flow sensing assembly disclosed herein can be used in other applications outside an anesthesia machine. In these aspects, it is contemplated that the sensing assembly 20 can be provided as a freestanding component of a system for measuring gaseous agent concentration within a gas or fluid line, such as, for example and without limitation, an application in which the sensing assembly functions as an anesthetic agent concentration sensing assembly. Optionally, the sensing assembly can comprise a housing 26 that defines an inlet opening 29a, an outlet opening 29b, and a central channel 28 extending between the inlet and outlet openings. In use, it is contemplated that the central channel 28 can be configured to be positioned in alignment and fluid communication with a gas flow line. In exemplary aspects, the sensors of the flow sensing assembly as disclosed herein can be positioned in communication with the central channel 28 to permit measurement of properties within a continuous gas flow pathway from the gas flow line through the central channel. For example, in some optional aspects, the flow sensing assembly can comprise a thermal sensor and a differential pressure sensor that are positioned in communication with the central channel and configured to produce respective measurement signals indicative of changes in temperature and pressure within the central channel. Optionally, as further disclosed herein, the sensing assembly 20 can be provided within processing circuitry that is communicatively coupled to the sensor(s) (e.g., the thermal sensor and the differential pressure sensor). In use, the processing circuitry can be configured to: receive the measurement signals from the thermal sensor and the differential pressure sensor; and correlate the measured changes in temperature and pressure to a change in the velocity of gas flow and to the concentration of a gaseous anesthetic agent within the gas flow line.

In operation, a method of using the system can comprise delivering a gaseous anesthetic agent to a breathing circuit. Optionally, the processing circuitry can receive an input indicative of the type of anesthetic agent(s) delivered to the breathing circuit. Such an input can be provided manually by a user through a user interface or can be received by a separate computing device. The method can further comprise circulating gas within the breathing circuit. Additionally, the method can further comprise using the flow sensing assembly to produce at least one measurement signal indicative of the concentration of the gaseous anesthetic agent within the breathing circuit. Optionally, when the system comprises a reflector assembly, the method can further comprise capturing gaseous anesthetic agent that exits the breathing circuit and returning the captured anesthetic agent to the breathing circuit.

Optionally, the blower assembly (e.g., electrical blower motor) can have a tachometer or other sensor for providing an output (e.g., revolutions per minute) that can be correlated to gas flow. During calibration, it is contemplated that the output of the tachometer or other sensor can be correlated to the output of the flow sensing assembly . It is contemplated that the outputs of the tachometer (or other sensor) and the flow sensing assembly can be affected by the concentration of volatile anesthetic agent (AA) within the breathing circuit. However, they are affected in different ways. In operation, the flow sensing assembly (which can optionally comprise a hot-wire anemometer) can be calibrated to estimate the tachometer (or other sensor) reading at 0% AA concentration. The difference between the tachometer (or other sensor) reading and the output of the flow sensing assembly can be used to indicate the AA concentration in the breathing circuit. It is contemplated that this approach can be generalized to using a different type of flow sensor, or to using two different types of flow sensors (i.e., instead of using the tachometer reading, use a flow sensor of a different principle than the other flow sensor). It is further contemplated that this approach can be further generalized by using additional sensors to compensate for the effects of humidity, temperature, or pressure in the breathing circuit. Thus, it is contemplated that the disclosed system can include any combination of sensors that permits realization of these calibration/estimation differences. For example, the disclosed methods can be based on linear regressions, nonlinear regressions, multi-variate regressions, or other approaches for estimating multi-variate relationships (e.g., wavelets, artificial neural networks, lookup tables, models of the underlying physics). In exemplary aspects, the disclosed systems and methods can permit monitoring of the effects of transitioning between different flow types (e.g., laminar vs. turbulent) based on the size of the breathing circuit and/or the flow in the breathing circuit.

Optionally, it is contemplated that the processor can determine an estimate of agent concentration and flow based on velocity from a using an empirical linear regression model based on the outputs received from a hot-wire anemometer or tachometer, as well as the measured differential pressure difference. Additionally, or alternatively, the processor can make use of look-up tables that can be referenced to correlate measured parameters (e.g., the outputs of the disclosed sensors) to agent concentrations and flow rates. Regardless of the specific method used to estimate agent concentration or flow, the underlying principle is the use of a flow sensor that is concentration-dependent (e.g., a differential pressure sensor that changes with fluid density) and another sensor that is concentration-independent (e.g., a hot-wire anemometer that can be tuned to measure only flow, regardless of fluid density). When a differential pressure sensor is used, the cause for the pressure difference is Bernoulli's principle, which states that the pressure difference through an orifice is equal to fluid velocity x fluid density ($\Delta P = (1/2)\rho\Delta v$). Thus, if the pressure difference and the velocity are known, the processor can be used to determine fluid density, which is proportional to the concentrations of the carrier gas (less dense) and the anesthetic (more dense).

The disclosed systems can replace expensive anesthetic gas benches as an anesthetic agent monitor with a system that contains few additional hardware components. Additionally, the disclosed systems can obviate the need for a side-stream monitor by making the use of a mainstream carbon dioxide ($CO_2$) monitor economically feasible.

As one will appreciate, the disclosed anesthesia systems offer a number of significant advantages, and function in a fundamentally different way, in comparison to conventional anesthesia systems and machines. For example, the disclosed system can provide for closed-loop control of the concentration of anesthetic agent delivered to a patient. Additionally, the presence of an in-line charcoal filter within the breathing circuit can rapidly reduce agent concentration when necessary. Further, the disclosed systems provide for a decoupling of fresh gas flow from anesthetic agent delivery. In contrast, current anesthesia machines couple the delivery of anesthetic and oxygen such that the anesthetic and oxygen must be delivered at the same time. Thus, in these existing anesthesia machines, if the patient needs more anesthetic but the concentration of oxygen is acceptable, it is necessary to add both new anesthetic and oxygen. During use of the disclosed system, the presence of the vaporizer in the rebreathing circuit allows for the addition of anesthetic without the need for adding additional oxygen to the breathing circuit.

As will be appreciated by one skilled in the art, the disclosed devices, methods, and systems may take the form of an entirely hardware example an entirely software example, or an example combining software and hardware aspects. Furthermore, the methods and systems may take the form of a computer program product on a computer-readable storage medium having computer-readable program instructions (e.g., computer software) embodied in the storage medium. More particularly, the present methods and systems may take the form of web-implemented computer software. Any suitable computer-readable storage medium may be utilized including hard disks, CD-ROMs, optical storage devices, or magnetic storage devices.

Examples of the methods and systems are described below with reference to block diagrams and flowchart illustrations of methods, systems, apparatuses and computer program products. It will be understood that each block of the block diagrams and flowchart illustrations, and combinations of blocks in the block diagrams and flowchart illustrations, respectively, can be implemented by computer program instructions. These computer program instructions may be loaded onto a general purpose computer, special purpose computer, or other programmable data processing apparatus to produce a machine, such that the instructions which execute on the computer or other programmable data processing apparatus create a means for implementing the functions specified in the flowchart block or blocks.

These computer program instructions may also be stored in a computer-readable memory that can direct a computer or other programmable data processing apparatus to function in a particular manner, such that the instructions stored in the computer-readable memory produce an article of manufacture including computer-readable instructions for implementing the function specified in the flowchart block or blocks. The computer program instructions may also be loaded onto a computer or other programmable data processing apparatus to cause a series of operational steps to be performed on the computer or other programmable apparatus to produce a computer-implemented process such that the instructions that execute on the computer or other programmable apparatus provide steps for implementing the functions specified in the flowchart block or blocks.

Accordingly, blocks of the block diagrams and flowchart illustrations support combinations of means for performing the specified functions, combinations of steps for performing the specified functions and program instruction means for performing the specified functions. It will also be understood that each block of the block diagrams and flowchart illustrations, and combinations of blocks in the block diagrams and flowchart illustrations, can be implemented by special purpose hardware-based computer systems that perform the specified functions or steps, or combinations of special purpose hardware and computer instructions.

One skilled in the art will appreciate that provided herein is a functional description and that the respective functions can be performed by software, hardware, or a combination of software and hardware. In an exemplary aspect, the methods and systems can be implemented, at least in part, on a computing device 101 as illustrated in FIG. ID and described below. By way of example, the controller 30 can be a computing device 101, and the processor 32, 103 described herein can be part of the computing device 101 as illustrated in FIG. ID. Similarly, the methods and systems disclosed can utilize one or more computing devices (e.g., computers, smartphones, or tablets) to perform one or more functions in one or more locations.

FIG. ID is a block diagram illustrating an exemplary operating environment for performing at least a portion of the disclosed methods. This exemplary operating environment is only an example of an operating environment and is not intended to suggest any limitation as to the scope of use or functionality of operating environment architecture. Neither should the operating environment be interpreted as having any dependency or requirement relating to any one or combination of components illustrated in the exemplary operating environment.

The present methods and systems can be operational with numerous other general purpose or special purpose computing system environments or configurations. Examples of well-known computing systems, environments, and/or configurations that can be suitable for use with the systems and methods comprise, but are not limited to, personal computers, server computers, laptop devices, and multiprocessor systems. Additional examples comprise set top boxes, programmable consumer electronics, network PCs, minicomputers, mainframe computers, distributed computing environments that comprise any of the above systems or devices, and the like.

The processing of the disclosed methods and systems can be performed by software components. The disclosed systems and methods can be described in the general context of computer-executable instructions, such as program modules, being executed by one or more computers or other devices. Generally, program modules comprise computer code, routines, programs, objects, components, data structures, etc., that perform particular tasks or implement particular abstract data types. The disclosed methods can also be practiced in grid-based and distributed computing environments where tasks are performed by remote processing devices that are linked through a communications network. In a distributed computing environment, program modules can be located in both local and remote computer storage media including memory storage devices.

Further, one skilled in the art will appreciate that the systems and methods disclosed herein can be implemented via a general-purpose computing device in the form of a computing device 101. The components of the computing device 101 can comprise, but are not limited to, one or more processors or processing units 103, a system memory 112, and a system bus 113 that couples various system components including the processor 103 to the system memory 112. In the case of multiple processing units 103, the system can utilize parallel computing.

The system bus 113 represents one or more of several possible types of bus structures, including a memory bus or memory controller, a peripheral bus, an accelerated graphics port, and a processor or local bus using any of a variety of bus architectures. By way of example, such architectures can comprise an Industry Standard Architecture (ISA) bus, a Micro Channel Architecture (MCA) bus, an Enhanced ISA (EISA) bus, a Video Electronics Standards Association (VESA) local bus, an Accelerated Graphics Port (AGP) bus, and a Peripheral Component Interconnects (PCI), a PCI-Express bus, a Personal Computer Memory Card Industry Association (PCMCIA), Universal Serial Bus (USB) and the like. The bus 113, and all buses specified in this description can also be implemented over a wired or wireless network connection and each of the subsystems, including the processor 103, a mass storage device 104, an operating system 105, control processing software 106, control processing data 107, a network adapter 108, system memory 112, an Input/Output Interface 110, a display adapter 109, a display device 111, and a human machine interface 102, can be contained within one or more remote computing devices 114a,b,c at physically separate locations, connected through buses of this form, in effect implementing a fully distributed system.

The computing device 101 typically comprises a variety of computer readable media. Exemplary readable media can be any available media that is accessible by the computing device 101 and comprises, for example and not meant to be limiting, both volatile and non-volatile media, removable and non-removable media. The system memory 112 comprises computer readable media in the form of volatile memory, such as random access memory (RAM), and/or non-volatile memory, such as read only memory (ROM). The system memory 112 typically contains data such as control processing data 107 and/or program modules such as operating system 105 and control processing software 106 that are immediately accessible to and/or are presently operated on by the processing unit 103.

In another aspect, the computing device 101 can also comprise other removable/non-removable, volatile/non-volatile computer storage media. By way of example, a mass storage device 104 can provide non-volatile storage of computer code, computer readable instructions, data structures, program modules, and other data for the computing device 101. For example and not meant to be limiting, a mass storage device 104 can be a hard disk, a removable magnetic disk, a removable optical disk, magnetic cassettes or other magnetic storage devices, flash memory cards, CD-ROM, digital versatile disks (DVD) or other optical storage, random access memories (RAM), read only memories (ROM), electrically erasable programmable read-only memory (EEPROM), and the like.

Optionally, any number of program modules can be stored on the mass storage device 104, including by way of example, an operating system 105 and control processing software 106. Each of the operating system 105 and control processing software 106 (or some combination thereof) can comprise elements of the programming and the control processing software 106. Control processing data 107 can also be stored on the mass storage device 104. Control processing data 107 can be stored in any of one or more databases known in the art. Examples of such databases comprise, DB2®, Microsoft® Access, Microsoft® SQL Server, Oracle®, mySQL, PostgreSQL, and the like. The databases can be centralized or distributed across multiple systems.

In another aspect, the user can enter commands and information into the computing device 101 via an input device, such as, without limitation, a keyboard, pointing device (e.g., a "mouse"), a microphone, a joystick, a scanner, tactile input devices such as gloves, and other body coverings, and the like. These and other input devices can be connected to the processing unit 103 via a human machine interface that is coupled to the system bus 113, but can be connected by other interface and bus structures, such as a parallel port, game port, an IEEE 1394 Port (also known as a Firewire port), a serial port, a universal serial bus (USB), or an Intel® Thunderbolt.

Optionally, in exemplary aspects, the processor 32, 103 of the controller 30 disclosed herein can receive manual inputs from a user or other individual supervising the delivery of anesthetic agents to a patient. Such manual inputs can correspond to an identity of a delivered agent, desired agent concentrations or concentration limits, desired flow rates, desired flow pathway configurations (including instructions regarding opening, closing, or adjusting of the positions of any valves disclosed herein), or patient information (physical condition, age, weight, and the like). It is further contemplated that the processor 32, 103 can be communicatively coupled to other components, such as a heart rate monitor or other monitoring device that provides physiological feedback (e.g. heart rate) or other parameter measurements to the processor 32, 103. It is still further contemplated that the processor 32, 103 can be communicatively coupled to a memory as further disclosed herein that stores a pre-set profile corresponding to the patient or to a particular anesthesia administration protocol. In operation, the processor 32, 103 can make use of these instructions to provide a customized anesthesia delivery profile for the patient and ensure that any adjustments to the anesthesia delivery parameters or system configuration are consistent with the instructions.

In yet another aspect, the display device 111 can also be connected to the system bus 113 via an interface, such as a display adapter 109. It is contemplated that the computing device 101 can have more than one display adapter 109 and the computing device 101 can have more than one display device 111. For example, a display device can be a monitor, an LCD (Liquid Crystal Display), an OLED (Organic Light Emitting Diode), or a projector. In addition to the display device 111, other output peripheral devices can comprise components such as speakers (not shown) and a printer (not shown) which can be connected to the computing device 101 via Input/Output Interface 110. Any step and/or result of the methods can be output in any form to an output device. Such output can be any form of visual representation, including, but not limited to, textual, graphical, animation, audio, tactile, and the like. The display 111 and computing device 101 can be part of one device, or separate devices.

The computing device 101 can operate in a networked environment using logical connections to one or more remote computing devices 114a,b,c. By way of example, a remote computing device can be a personal computer, portable computer, smartphone, a tablet, a server, a router, a network computer, a peer device or other common network node, and so on. In exemplary aspects, a remote computing device can be operated by a clinician involved with the administration (or supervision of the administration) of anesthesia to the patient. Logical connections between the computing device 101 and a remote computing device 114a,b,c can be made via a network 115, such as a local area network (LAN) and/or a general wide area network (WAN). Such network connections can be through a network adapter 108. A network adapter 108 can be implemented in both wired and wireless environments. Such networking environments are conventional and commonplace in dwellings, offices, enterprise-wide computer networks, intranets, and the Internet.

For purposes of illustration, application programs and other executable program components such as the operating system 105 are illustrated herein as discrete blocks, although it is recognized that such programs and components reside at various times in different storage components of the computing device 101, and are executed by the data processor(s) of the computer. An implementation of control processing software 106 can be stored on or transmitted across some form of computer readable media. Any of the disclosed methods can be performed by computer readable instructions embodied on computer readable media. Computer readable media can be any available media that can be accessed by a computer. By way of example and not meant to be limiting, computer readable media can comprise "computer storage media" and "communications media." "Computer storage media" comprise volatile and nonvolatile, removable and non-removable media implemented in any methods or technology for storage of information such as computer readable instructions, data structures, program modules, or other data. Exemplary computer storage media comprises, but is not limited to, RAM, ROM, EEPROM, solid state, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computer.

The methods and systems can employ Artificial Intelligence techniques such as machine learning and iterative learning. Examples of such techniques include, but are not limited to, expert systems, case based reasoning, Bayesian networks, behavior based AI, neural networks, fuzzy systems, evolutionary computation (e.g. genetic algorithms), swarm intelligence (e.g. ant algorithms), and hybrid intelligent systems (e.g. Expert inference rules generated through a neural network or production rules from statistical learning).

The above-described system components may be local to one of the devices (e.g., a computing device, such as a tablet or smartphone) or remote (e.g. servers in a remote data center, or "the cloud"). In exemplary aspects, it is contemplated that many of the system components can be provided in a "cloud" configuration.

**EXPERIMENTAL EXAMPLES**

The presently described technology and its advantages will be better understood by reference to the following examples. These examples are provided to describe non-exhaustive embodiments of the present technology. By providing these examples, the scope of the presently described and claimed technology is not limited in spirit or scope. It will be understood by those skilled in the art that the full scope of the presently described technology encompasses at least the subject matter defined by the claims appending this specification, and any alterations, modifications, derivatives, combinations, or equivalents of those claims.

Example One

METHODS

A hot wire anemometer (AWM700 Series Airflow Sensor, Honeywell, Golden Valley, MN) was placed inline of a fresh gas flow outlet at known flow rates ranging from 2-12 liters per minute (measured using VT-Plus Gas Flow Analyzer, Fluke Corp., Everett, WA). This gas flow contained 0-3.25% isoflurane (measured using an infrared gas bench, Datex-Ohmeda, Helsinki, Finland) and a balance of oxygen. Baseline measurements were used to calibrate the hot wire anemometer voltage at 0% isoflurane. When isoflurane was introduced into the system, deviations from this baseline voltage were attributed to changes in the isoflurane concentration.

RESULTS

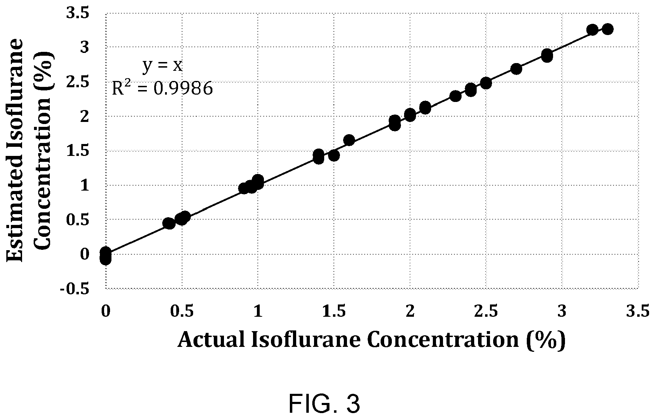

A total of forty-two isoflurane estimations were obtained. The estimated isoflurane concentration was highly correlative with the measured Datex-Ohmeda Gas Analyzer isoflurane concentration with an average error of 0% isoflurane and standard deviation of 0.04% isoflurane (FIG. 3).

These preliminary results suggest that hot wire anemometry can be an effective mainstream method to measure volatile anesthetic gas concentration, thereby allowing the creation of a cheaper anesthetic machine with improved feedback control properties.

Example Two

METHODS

An air-oxygen mixture was circulated through a custom rebreathing circuit using a radial blower (U51DL-012KK-4 Miniature Radial Blower with Integrated Electronics, Micronel, Tagelswangen, Switzerland) with flows ranging from 10-70 liters per minute (measured using the integrated electronics of the radial blower, independently verified using a VT-Plus Gas Flow Analyzer, Fluke Corp., Everett, WA). Isoflurane (Piramal Healthcare Limited, Andhra Pradesh, India) was introduced to the rebreathing circuit with a custom vaporizer, at concentration ranging from 0-3.5% measured using a standard side stream infrared gas bench (Datex-Ohmeda, Helsinki, Finland). Placed inline of this gas flow was a hot wire anemometer (AWM700 Series Airflow Sensor, Honeywell, Golden Valley, MN). Baseline measurements at all flow rates were used to calibrate the hot wire anemometer. As isoflurane was introduced to the rebreathing circuit, deviations from this baseline were attributed to changes in the isoflurane concentration. A model was generated to estimate the isoflurane concentration and compared to actual isoflurane concentrations.

RESULTS

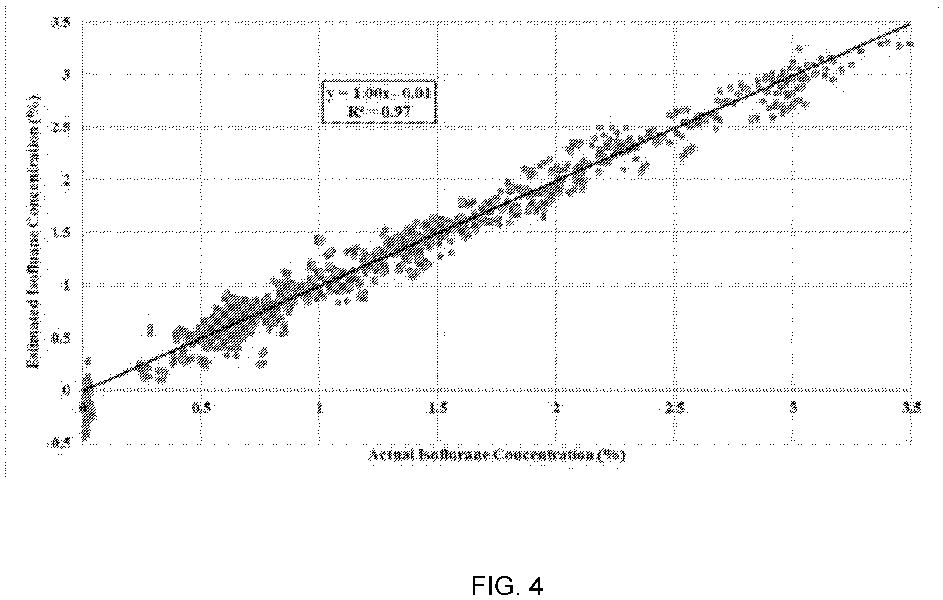

Isoflurane concentration estimations were highly correlative to measured isoflurane concentrations (R2=0.97). In a sample size of N=1560, the mean error was -0.01% isoflurane with a standard deviation of 0.13% isoflurane (Figure 4).

Results show that monitoring heat capacity is an adequate, low-cost, and robust method to measure volatile anesthetic gas concentration.

Example Three

METHODS

A 5 liter per minute flow of oxygen and 5% isoflurane (Piramal Healthcare Limited, Andhra Pradesh, India) was delivered through a cylindrical vessel containing approximately 40 grams of activated charcoal (Oxpure 1220C-75, Oxbow Activated Carbon, West Palm Beach, FL) until 0.5% isoflurane pushed through the charcoal. Flow was then reversed through the vessel at 2 liters per minute with pure oxygen, and the concentration of isoflurane leaving the vessel was monitored using a standard side stream infrared gas bench (Datex-Ohmeda, Helsinki, Finland). Additionally, a smaller vessel containing 10 grams of activated charcoal was saturated and placed medially of a rebreathing Y-piece and test lung. The test lung was then driven using a ventilator at 0% isoflurane and the concentration of isoflurane was monitored. A control vessel containing inert non-porous beads was used in both studies.

RESULTS

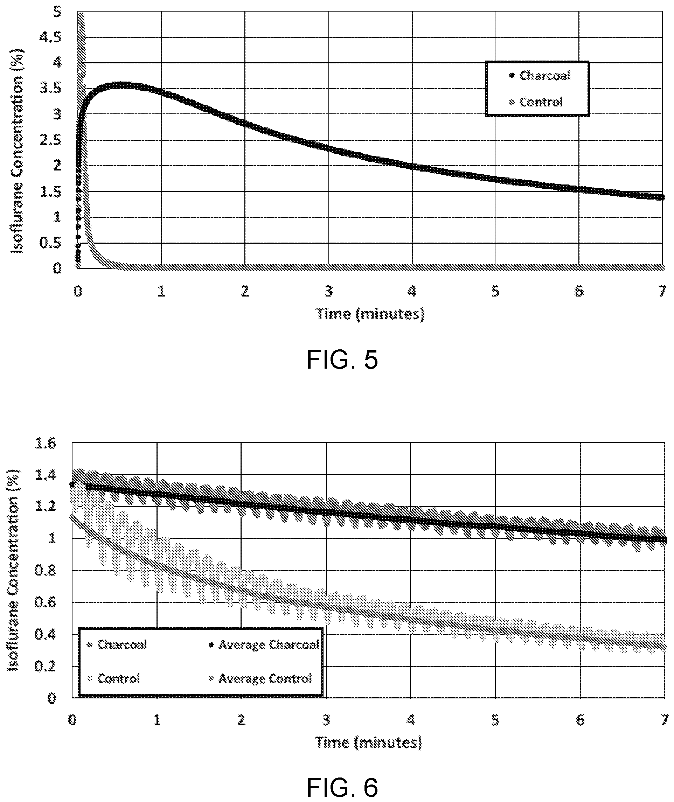

Isoflurane was released at concentrations suitable for anesthesia maintenance for approximately 10 minutes. Once saturated, the activated charcoal had absorbed approximately 60% of its total weight in isoflurane, and was capable of repeatedly reflecting 10% of its total weight in isoflurane or about 3.2 mL of liquid isoflurane. For the larger vessel, (approximately 40 grams of activated charcoal), the volume of isoflurane that was capable of being reflected can achieve anesthesia maintenance at 1 MAC for 1 hour at a fresh gas flow rate of 1 liter per minute. (See FIGS. 5-6).

These results show that activated charcoal can be a feasible material in reflecting and conserving anesthetic gases.

Example Four

METHODS

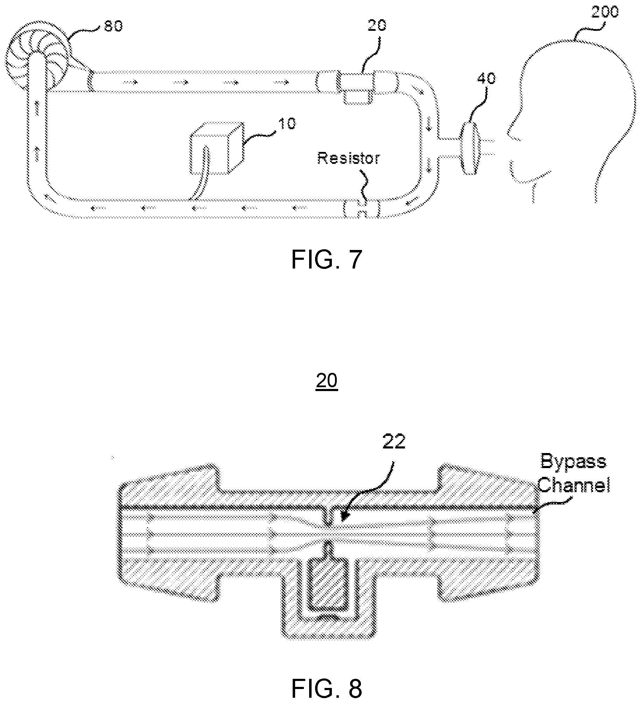

An exemplary configuration of an anesthesia system utilizing a combination differential pressure and thermal flow sensor in conjunction with a radial blower motor was used to determine isoflurane gas concentrations in a test breathing circuit, as shown in FIG. 7. A radial blower can provide a continuous gas flow as well as the changes in pressure to ventilate a test lung. The radial blower can also provide instantaneous flow rate, which can be later used to determine gas concentration. The anesthetic gas sensor, a combination of a differential pressure and thermal flow sensor, can determine anesthetic concentrations by sensing the changes in gas density. The gas density can be determined by comparing the sensed flow rate to the precise flow rate provided by the radial blower. A heat and moisture exchanger ("HME") can be placed in front of the patient, or rather a test lung, to isolate the patient from the rebreathing circuit. A fluid resistor can allow for the radial blower to not only provide different flow rates, but also a range of standard clinical pressures to ventilate the test lung. A custom vaporizer can introduce anesthetic gas into the rebreathing circuit. The flow sensor can comprise a bypass channel, as shown in FIG. 8, which depends on the differential pressure generated by an orifice and a thermal flow sensor within that channel. Bernoulli's equation (provided below) shows that if the fluid velocity is known (controlled from the radial blower), then the density of the gas can be determined by measuring the pressure drop through a constriction. Bernoulli's equation is as follows:

$$\frac{1}{2}\rho v_1^2 + P_1 = \frac{1}{2}\rho v_2^2 + P_2$$

$$\Delta P = \frac{1}{2}\rho \Delta v^2$$

RESULTS

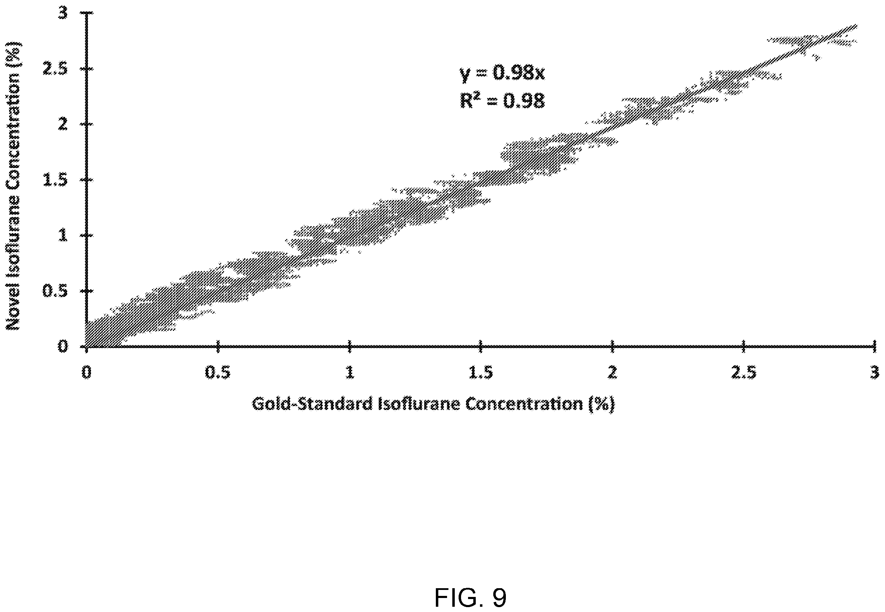

While a test lung was ventilated under standard clinical pressures, the anesthetic gas concentration was measured alongside a standard infrared gas bench. The mean difference in measured isoflurane concentration was -0.025% isoflurane with a standard deviation of 0.091% isoflurane (FIG. 9). The preliminary data shows that the described method of measuring anesthetic gas concentration can also be used with a hot-wire anemometer as an alternative to the radial blower.

The results show that use of an anesthesia system that utilizes a combination of a differential pressure and thermal flow sensor in conjunction with a radial blower motor can provide an accurate, low-cost, versatile, and portable method to measure anesthetic gas concentration.

**Claims**

1. An anesthesia system (100) defining a breathing circuit (2) and comprising:

a vaporizer (10) configured to deliver a gaseous anesthetic agent to the breathing circuit;
a flow sensing assembly (20) configured to produce a measurement signal indicative of a velocity of gas flow within the breathing circuit; and
a processing circuitry (32) that is communicatively coupled to the flow sensing assembly, wherein the processing circuitry is configured to receive the measurement signal produced by the flow sensing assembly and to determine the concentration of the gaseous anesthetic agent within the breathing circuit,
**characterised by** the flow sensing assembly comprising a thermal sensor (22) and a differential pressure sensor (24),
wherein the thermal and differential pressure sensors are configured to produce at least one measurement signal indicative of changes in temperature and pressure within the breathing circuit, and wherein the processing circuitry is configured to correlate the measured changes in temperature and pressure to a change in the velocity of gas flow and to the concentration of the gaseous anesthetic agent within the breathing circuit.

2. The system of claim 1, further comprising a patient line (5) positioned in fluid communication with the breathing circuit, wherein the patient line is located downstream of the vaporizer.

3. The system of claim 2, wherein the patient line comprises an air-moisture exchanger.

4. The system of claim 2, further comprising:
a charcoal filter (50) positioned in fluid communication with the breathing circuit between the vaporizer and the patient line.

5. The system of claim 4, further comprising a carbon dioxide scrubber (60) positioned within the breathing circuit and between the patient line and the flow sensor.

6. The system of claim 5, further comprising a reservoir (70) positioned in fluid communication with the breathing circuit between the patient line and the carbon dioxide scrubber.

7. The system of claim 5, further comprising a bypass line configured to permit selective bypassing of the carbon dioxide scrubber.

8. The system of any one of the preceding claims, further comprising a blower assembly (80) configured to circulate gas within the breathing circuit, wherein the blower assembly comprises a blower motor that is communicatively coupled to the processing circuit-

**9.** The system of claim 5, further comprising:
a gas inlet-scavenge line (6) positioned in fluid communication with the breathing circuit, wherein the gas inlet-scavenge line is positioned between the carbon dioxide scrubber and the flow sensor.

**10.** The system of claim 9, further comprising a reflector assembly (90) positioned in selective fluid communication with the breathing circuit through the gas inlet-scavenge line, wherein the reflector assembly comprises an anesthetic agent reflector configured to capture gaseous anesthetic agent that exits the breathing circuit through the gas inlet-scavenge line, and wherein the reflector assembly is configured to return the captured anesthetic agent to the breathing circuit.

**11.** The system of claim 10, wherein the reflector assembly further comprises first and second fluid control valves (94 and 96) positioned on opposing upstream and downstream sides of the anesthetic agent reflector, wherein the second fluid control valve (96) is positioned between the anesthetic agent reflector and the breathing circuit, and wherein the first and second fluid control valves are communicatively coupled to the processing circuitry.

**12.** The system of claim 11, further comprising:

a gas inlet (7) positioned in fluid communication with the first fluid control valve, wherein the gas inlet is configured to receive fresh gas flow from a gas source; and
a scavenge outlet (8) positioned in fluid communication with the first fluid control valve.

**13.** The system of claim 12, wherein the first and second fluid control valves are moveable about and between respective first and second positions, and wherein the processing circuitry is configured to selectively move the first and second fluid control valves about and between the first position and the second position, wherein the first position of the first fluid control valve corresponds to a gas-receiving position in which fresh gas flow from the gas inlet is allowed to pass through the first fluid control valve, wherein the second position of the first fluid control valve corresponds to a scavenge position in which fresh gas flow is blocked from entering the first fluid control valve but the first fluid control valve permits passage of gas into the scavenger outlet, wherein the first position of the second fluid control valve corresponds to an open position that permits gas flow through the valve, and wherein the second position of the second fluid control valve corresponds to a closed position

that blocks gas flow through the valve.

**14.** The system of claim 13, wherein the processing circuitry is configured to move the first and second valves to define an open breathing circuit configuration, a closed breathing circuit configuration, or a partially closed breathing circuit configuration, wherein in the open breathing circuit configuration, the first and second valves are respectively positioned in the gas-receiving and open positions, wherein in the partially open breathing circuit configuration, the first valve is positioned in the scavenge position and the second valve is positioned in the open position, and wherein in the closed breathing circuit configuration, the second valve is positioned in the closed position.

**15.** The system of claim 1, wherein the thermal sensor comprises a hot-wire anemometer having a heated resistive wire, wherein the hot-wire anemometer is configured to produce a measurement signal indicative of a voltage required to maintain a temperature of the heated resistive wire as gas in communication with the flow sensor flows within the breathing circuit, and wherein the processing circuitry is configured to correlate the required voltage to a change in the velocity of gas flow and to the concentration of the gaseous anesthetic agent within the breathing circuit.

**Patentansprüche**

**1.** Ein Anästhesiesystem (100), das einen Atmungskreislauf (2) definiert und Folgendes umfasst:

einen Verdampfer (10), der so konfiguriert ist, dass er ein gasförmiges Anästhesiemittel an den Atmungskreislauf abgibt;
eine Strömungssensoranordnung (20), die so konfiguriert ist, dass sie ein Messsignal erzeugt, das eine Geschwindigkeit der Gasströmung innerhalb des Atmungskreislaufs anzeigt; und
eine Verarbeitungsschaltung (32), die kommunikativ mit der Strömungssensoranordnung gekoppelt ist, wobei die Verarbeitungsschaltung so konfiguriert ist, dass sie das von der Strömungssensoranordnung erzeugte Messsignal empfängt und die Konzentration des gasförmigen Narkosemittels im Atmungskreislauf bestimmt;
**dadurch gekennzeichnet, dass** die Strömungssensoranordnung einen thermischen Sensor (22) und einen Differenzdrucksensor (24) umfasst, wobei der thermische Sensor und der Differenzdrucksensor so konfiguriert sind, dass sie mindestens ein Messsignal erzeugen, das Änderungen der Temperatur und des Drucks innerhalb des Atmungskreislaufs anzeigt, und wobei die Verarbeitungsschaltung so konfiguriert ist, dass sie die gemessenen Änderungen der Temperatur und des Drucks mit einer Änderung der Geschwindigkeit des Gasflusses und der Konzentration des gasförmigen Anästhetikums innerhalb des Atmungskreislaufs korreliert.

2. System nach Anspruch 1, das ferner eine Patientenleitung (5) umfasst, die in Flussverbindung mit dem Atmungskreislauf steht, wobei die Patientenleitung stromabwärts des Verdampfers angeordnet ist.

3. System nach Anspruch 2, wobei die Patientenleitung einen Luft-Feuchtigkeits-Austauscher umfasst.

4. Das System nach Anspruch 2, ferner umfassend: einen Aktivkohlefilter (50), der in Flussverbindung mit dem Atmungskreislauf zwischen dem Verdampfer und der Patientenleitung angeordnet ist.

5. System nach Anspruch 4, das ferner einen Kohlendioxid-Wäscher (60) umfasst, der innerhalb des Atmungskreislaufs und zwischen der Patientenleitung und dem Strömungssensor angeordnet ist.

6. Das System nach Anspruch 5 umfasst ferner ein Reservoir (70), das in Flussverbindung mit dem Atmungskreislauf zwischen der Patientenleitung und dem Kohlendioxidwäscher angeordnet ist.

7. System nach Anspruch 5, das ferner eine Umgehungsleitung umfasst, die so konfiguriert ist, dass sie eine selektive Umgehung des Kohlendioxid-Wäschers ermöglicht.

8. System nach einem der vorhergehenden Ansprüche, das ferner eine Gebläseanordnung (80) umfasst, die so konfiguriert ist, dass sie Gas innerhalb des Atmungskreislaufs zirkulieren lässt, wobei die Gebläseanordnung einen Gebläsemotor umfasst, der kommunikativ mit der Verarbeitungsschaltung gekoppelt ist, und wobei die Gebläseanordnung stromaufwärts des Strömungssensors angeordnet ist.

9. Das System nach Anspruch 5, ferner umfassend: eine Gaseinlass-Spülleitung (6), die in Flussverbindung mit dem Atmungskreislauf positioniert ist, wobei die Gaseinlass-Spülleitung zwischen dem Kohlendioxid-Wäscher und dem Strömungssensor positioniert ist.

10. System nach Anspruch 9, das ferner eine Reflektoranordnung (90) umfasst, die durch die Gaseinlass-Spülleitung in selektiver Flussverbindung mit dem Atmungskreislauf positioniert ist, wobei die Reflektoranordnung einen Narkosemittelreflektor umfasst, der so konfiguriert ist, dass er gasförmiges Narkosemittel einfängt, das den Atmungskreislauf durch die Gaseinlass-Spülleitung verlässt, und wobei die Reflektoranordnung so konfiguriert ist, dass sie das eingefangene Narkosemittel in den Atmungskreislauf zurückführt.

11. System nach Anspruch 10, wobei die Reflektoranordnung ferner ein erstes und ein zweites Flusssteuerventil (94 und 96) umfasst, die an gegenüberliegenden stromaufwärtigen und stromabwärtigen Seiten des Narkosemittelreflektors positioniert sind, wobei das zweite Flusssteuerventil (96) zwischen dem Narkosemittelreflektor und dem Atmungskreislauf positioniert ist und wobei das erste und das zweite Flusssteuerventil kommunikativ mit der Verarbeitungsschaltung gekoppelt sind.

12. Das System nach Anspruch 11, ferner umfassend:

einen Gaseinlass (7), der in Flussverbindung mit dem ersten Flusssteuerventil angeordnet ist, wobei der Gaseinlass so konfiguriert ist, dass er einen Frischgasstrom von einer Gasquelle empfängt; und
einen Spülungsauslass (8), der in Flussverbindung mit dem ersten Flusssteuerventil steht.

13. System nach Anspruch 12, wobei das erste und das zweite Flusssteuerventil um und zwischen einer ersten bzw. einer zweiten Position bewegbar sind, und wobei die Verarbeitungsschaltung so konfiguriert ist, dass sie das erste und das zweite Flusssteuerventil selektiv um und zwischen der ersten Position und der zweiten Position bewegt, wobei die erste Position des ersten Flusssteuerventils einer Gasaufnahmeposition entspricht, in der ein Frischgasstrom von dem Gaseinlass durch das erste Flusssteuerventil hindurchgelassen wird, wobei die zweite Position des ersten Flusssteuerventils einer Spülposition entspricht, in der der Eintritt von Frischgasstrom in das erste Flusssteuerventil blockiert ist, aber das erste Flusssteuerventil den Durchgang von Gas in den Spülauslass erlaubt, wobei die erste Position des zweiten Flusssteuerventils einer offenen Position entspricht, die einen Gasstrom durch das Ventil erlaubt, und wobei die zweite Position des zweiten Flusssteuerventils einer geschlossenen Position entspricht, die den Gasstrom durch das Ventil blockiert.

14. System nach Anspruch 13, wobei die Verarbeitungsschaltung so konfiguriert ist, dass sie das erste und das zweite Ventil bewegt, um eine offene Atmungskreislaufkonfiguration, eine geschlossene Atmungskreislaufkonfiguration oder eine teilweise geschlossene Atmungskreislaufkonfiguration zu definieren, wobei in der offenen Atmungskreislaufkonfiguration das erste und das zweite Ventil jeweils in der Gasaufnahmeposition und der offenen Position positioniert sind, wobei in der teilweise offenen Atmungskreislaufkonfiguration das erste Ventil in der Spülposition und das zweite Ventil in der offenen Position positioniert ist, und wobei in der geschlossenen Atmungskreislaufkonfiguration das zweite Ventil in der geschlossenen Position positioniert ist.

15. System nach Anspruch 1, wobei der thermische Sensor ein Hitzedrahtanemometer mit einem beheizten Widerstandsdraht umfasst, wobei das Hitzedrahtanemometer so konfiguriert ist, dass es ein Messsignal erzeugt, das eine Spannung anzeigt, die erforderlich ist, um eine Temperatur des beheizten Widerstandsdrahtes aufrechtzuerhalten, wenn Gas, das mit dem Strömungssensor in Verbindung steht, innerhalb des Atmungskreislaufs strömt, und wobei die Verarbeitungsschaltung so konfiguriert ist, dass sie die erforderliche Spannung mit einer Änderung der Geschwindigkeit des Gasflusses und der Konzentration des gasförmigen Anästhetikums innerhalb des Atmungskreislaufs korreliert.

**Revendications**

1. Système d'anesthésie (100) définissant un circuit respiratoire (2) et comprenant :

   un vaporisateur (10) configuré pour délivrer un agent anesthésique gazeux au circuit respiratoire ;
   un ensemble de détection d'écoulement (20) configuré pour produire un signal de mesure indicatif d'une vitesse d'écoulement de gaz dans le circuit respiratoire ; et

   un circuit de traitement (32) qui est couplé de manière communicative à l'ensemble de détection d'écoulement, dans lequel le circuit de traitement est configuré pour recevoir le signal de mesure produit par l'ensemble de détection d'écoulement et pour déterminer la concentration de l'agent anesthésique gazeux dans le circuit respiratoire,
   **caractérisé en ce que** l'ensemble de détection d'écoulement comprend un capteur thermique (22) et un capteur de pression différentielle (24), dans lequel les capteurs thermiques et de pression différentielle sont configurés pour produire au moins un signal de mesure indiquant des changements de température et de pression à l'intérieur du circuit respiratoire, et dans lequel le circuit de traitement est configuré pour corréler les changements mesurés de température et de pression à un changement de la vitesse d'écoulement du gaz et à la concentration de l'agent anesthésique gazeux à l'intérieur du circuit respiratoire.

2. Le système de la revendication 1, comprenant en outre une ligne de patient (5) positionnée en communication fluide avec le circuit respiratoire, dans lequel la ligne de patient est située en aval du vaporisateur.

3. Le système de la revendication 2, dans lequel la ligne du patient comprend un échangeur air-humidité.

4. Le système de la revendication 2, comprenant en outre :
   un filtre à charbon (50) positionné en communication fluidique avec le circuit respiratoire entre le vaporisateur et la ligne patient.

5. Le système de la revendication 4, comprenant en outre un épurateur de dioxyde de carbone (60) positionné à l'intérieur du circuit respiratoire et entre la ligne du patient et le capteur de débit.

6. Le système de la revendication 5, comprenant en outre un réservoir (70) positionné en communication fluidique avec le circuit respiratoire entre la ligne du patient et l'épurateur de dioxyde de carbone.

7. Le système de la revendication 5, comprenant en outre une ligne de dérivation configurée pour permettre la dérivation sélective de l'épurateur de dioxyde de carbone.

8. Le système de l'une quelconque des revendications précédentes, comprenant en outre un ensemble de soufflerie (80) configuré pour faire circuler du gaz à l'intérieur du circuit respiratoire, dans lequel l'ensemble de soufflerie comprend un moteur de soufflerie qui est couplé de manière communicative aux circuits de traitement, et dans lequel l'ensemble de soufflerie est positionné en amont du capteur de débit.

9. Le système de la revendication 5, comprenant en outre :
   une ligne d'admission et de captation de gaz (6) positionnée en communication fluidique avec le circuit respiratoire, dans laquelle la ligne d'admission et de captation de gaz est positionnée entre l'épurateur de dioxyde de carbone et le capteur d'écoulement.

10. Le système de la revendication 9, comprenant en outre un ensemble réflecteur (90) positionné en communication fluidique sélective avec le circuit respiratoire par l'intermédiaire de la ligne d'admission et de collecte de gaz, dans lequel l'ensemble réflecteur comprend un réflecteur d'agent anesthésique configuré pour capturer l'agent anesthésique gazeux qui sort du circuit respiratoire par l'intermédiaire de la ligne d'admission et de collecte de gaz, et dans lequel l'ensemble réflecteur est configuré pour renvoyer l'agent anesthésique capturé vers le circuit respiratoire.

**11.** Le système selon la revendication 10, dans lequel l'ensemble réflecteur comprend en outre des première et seconde vannes de commande de fluide (94 et 96) positionnées sur des côtés amont et aval opposés du réflecteur d'agent anesthésique, dans lequel la seconde vanne de commande de fluide (96) est positionnée entre le réflecteur d'agent anesthésique et le circuit respiratoire, et dans lequel les première et seconde vannes de commande de fluide sont couplées de manière communicative au circuit de traitement.

**12.** Le système de la revendication 11, comprenant en outre :

une entrée de gaz (7) positionnée en communication fluidique avec la première vanne de commande de fluide, dans laquelle l'entrée de gaz est configurée pour recevoir un flux de gaz frais provenant d'une source de gaz ; et
une sortie de balayage (8) positionnée en communication fluidique avec la première vanne de commande de fluide.

**13.** Le système de la revendication 12, dans lequel les première et seconde vannes de commande de fluide sont mobiles autour et entre des première et seconde positions respectives, et dans lequel le circuit de traitement est configuré pour déplacer sélectivement les première et seconde vannes de commande de fluide autour et entre la première position et la seconde position, dans lequel la première position de la première vanne de commande de fluide correspond à une position de réception de gaz dans laquelle un écoulement de gaz frais provenant de l'entrée de gaz est autorisé à passer à travers la première vanne de commande de fluide, dans laquelle la seconde position de la première vanne de commande de fluide correspond à une position de balayage dans laquelle l'écoulement de gaz frais est empêché d'entrer dans la première vanne de commande de fluide mais la première vanne de commande de fluide permet le passage de gaz dans la sortie de balayage, dans laquelle la première position de la seconde vanne de commande de fluide correspond à une position ouverte qui permet l'écoulement de gaz à travers la vanne, et dans laquelle la seconde position de la seconde vanne de commande de fluide correspond à une position fermée qui bloque l'écoulement de gaz à travers la vanne.

**14.** Le système selon la revendication 13, dans lequel les circuits de traitement sont configurés pour déplacer les première et deuxième vannes pour définir une configuration de circuit respiratoire ouvert, une configuration de circuit respiratoire fermé, ou une configuration de circuit respiratoire partiellement fermé, dans lequel dans la configuration de circuit respiratoire ouvert, les première et deuxième vannes sont respectivement positionnées dans les positions de réception de gaz et ouverte, dans lequel dans la configuration de circuit respiratoire partiellement ouvert, la première vanne est positionnée dans la position d'évacuation et la deuxième vanne est positionnée dans la position ouverte, et dans lequel dans la configuration de circuit respiratoire fermé, la deuxième vanne est positionnée dans la position fermée.

**15.** Le système selon la revendication 1, dans lequel le capteur thermique comprend un anémomètre à fil chaud ayant un fil résistif chauffé, dans lequel l'anémomètre à fil chaud est configuré pour produire un signal de mesure indiquant une tension requise pour maintenir une température du fil résistif chauffé lorsque le gaz en communication avec le capteur d'écoulement s'écoule dans le circuit respiratoire, et dans lequel le circuit de traitement est configuré pour corréler la tension requise à un changement de la vitesse de l'écoulement du gaz et à la concentration de l'agent anesthésique gazeux dans le circuit respiratoire.

FIG. 1A

FIG. 1B

90
Reflector
Assembly

94
1st
Valve

96
2nd
Valve

110

Gas Source

80

Blower

20

Flow Sensor

3

Flow Pathway
Adjuster

30          Controller

32

Processor

34

Memory

FIG. 1C

FIG. 1D

FIG. 2

FIG. 3

FIG. 4

EP 3 451 921 B1

FIG. 5

FIG. 6

FIG. 7

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2011105934 A
- EP 0545567 A
- WO 2016065265 A
- US 2015209546 A

### Non-patent literature cited in the description

- **GATRAD AR et al.** *Anaesthesia,* 2007, vol. 62, 90-95
- **LIBBY, P. A. ; WAY, J.** Hot-wire probes for measuring velocity and concentration in helium-air mixtures. *AIAA Journal,* 1970, vol. 8 (5), 976-978